Europäisches Patentamt

European Patent Office

Office européen des brevets

(11) Numéro de publication: **0 307 274**
A1

## (12) DEMANDE DE BREVET EUROPEEN

(21) Numéro de dépôt: 88402150.2

(22) Date de dépôt: 24.08.88

(51) Int. Cl.⁴: **A 01 H 5/02**
A 01 G 7/00

(30) Priorité: 31.08.87 FR 8712100

(43) Date de publication de la demande:
15.03.89 Bulletin 89/11

(84) Etats contractants désignés:
BE CH DE GB LI NL SE

(71) Demandeur: **SOCIETE DE CREATION ET D'OBTENTION VEGETALE ET DE RECHERCHE BIOTECHNOLOGIQUE "SOCOTRA"**
31, rue Louis Bellan
FR-78890 Garancieres (FR)

(72) Inventeur: **Chaintron, Jean-Marc**
5, rue de Beconcelle
F-78910 Orgerus (FR)

(74) Mandataire: **Netter, André et al**
Cabinet NETTER 40, rue Vignon
F-75009 Paris (FR)

(54) Procédé de multiplication et de culture de plantes de la famille des broméliacées, et plantes ainsi obtenues.

(57) Procédé de multiplication et de culture de plantes de la famille des broméliacées pour obtenir des plantes ornementales en pot, selon lequel on fait croître la plante de manière qu'il se forme un pédoncule floral (16), on coupe ce pédoncule floral à un stade de développement où il porte un fruit (22) en formation et on fait enraciner le pédoncule floral (16) dans un pot (34) rempli de terre (36).

FIG. 2

Bundesdruckerei Berlin

EP 0 307 274 A1

## Description

### Procédé de multiplication et de culture de plantes de la famille des broméliacées, et plantes ainsi obtenues

L'invention concerne un procédé de multiplication et de culture de plantes de la famille des broméliacées, en particulier du genre ananas, et les plantes ainsi obtenues.

Les plantes de cette famille comprennent de nombreux genres, divisés en de multiples variétés, qui sont cultivées pour leurs fruits et/ou leurs fleurs, et cela à des fins de consommation et/ou d'ornementation.

Les plantes du genre ananas sont cultivées principalement pour leurs fruits à des fins de consommation, bien que certaines variétés, dont la consommation des fruits est de peu d'intérêt, soient cultivées en tant que plantes ornementales ou pour fournir des fleurs coupées à conserver en vases remplis d'eau.

Les plantes de la famille des broméliacées, en particulier du genre ananas, ont pour caractéristique commune de posséder une tige qui est pourvue de racines à sa base et qui présente des noeuds proches les uns des autres, d'où partent des feuilles. Au cours du développement de la plante, il se forme, à partir de la tige, et au-dessus des feuilles, un pédoncule floral encore appelé "tige inflorescencielle".

Ce pédoncule floral présente des caractéristiques morphologiques différentes de celles de la tige et, en particulier, présente des noeuds beaucoup plus espacés que ceux de la tige qu'il surmonte ou prolonge.

Au cours de son développement, ce pédoncule floral donne naissance à une fleur ou, plus exactement, à un groupe de fleurs dont l'ensemble des carpelles a l'aspect caractéristique du fruit auquel les fleurs donneront naissance.

Le fruit qui se développe à partir des fleurs est généralement surmonté d'une couronne de feuilles, encore appelée "toupet", dont les feuilles sont identiques à celles qui se forment à la base de la plante, mais sont d'une longueur plus petite.

Au cours de son développement, la plante donne naissance à des bourgeons qui croissent notamment à partir de la tige et dont certains forment des rejets.

Pour la reproduction, ce sont ces rejets qui sont après sélection séparés de la plante-mère pour permettre, par bouturage, la production d'autres plantes qui, à leur tour, donneront naissance à un fruit.

Lorsqu'une plante du genre ananas est cultivée à des fins de consommation, on recueille le fruit en coupant le pédoncule floral juste au-dessous du fruit, lorsque ce dernier est arrivé à maturité ou bien à un stade proche de la maturité.

Lorsqu'une plante du genre ananas est cultivée à des fins ornementales, on commercialise soit la plante en pot, soit la fleur coupée, c'est-à-dire le pédoncule floral surmonté de la fleur et/ou du fruit.

La plante en pot a pour inconvénient principal d'être particulièrement encombrante et difficile à transporter puisque son diamétre moyen est généralement de l'ordre de 50 à 70 cm.

Quant à la fleur coupée, elle a pour inconvénient principal d'avoir une durée de conservation limitée dans l'eau, au maximum de trois semaines à un mois, et d'être présentée difficilement dans un vase, compte tenu du poids relativement élevé du fruit qui surmonte le pédoncule floral.

C'est l'un des buts de l'invention de remédier aux inconvénients ci-dessus mentionnés en fournissant un procédé de multiplication et de culture de plantes de la famille des broméliacées qui permet d'obtenir des plantes ornementales d'un type nouveau.

La Demanderesse a constaté que, de manière surprenante, on pouvait faire multiplier les plantes de cette famille à partir d'une partie de la plante que, jusqu'à présent, l'on n'avait jamais utilisée ou même proposé d'utiliser pour la reproduction ou la multiplication.

Conformément à la technique antérieure, la plante est reproduite principalement, comme déjà indiqué précédemment, par bouturage de rejets sélectionnés suivant des critères bien précis.

Des tentatives, plus ou moins fructueuses, ont été tentées pour la reproduction de ces plantes à partir d'autres parties de la plante, par exemple de la tige ou encore des feuilles.

La Demanderesse a constaté qu'une multiplication de la plante était possible à partir du pédoncule floral, et cela dans des conditions bien définies.

Selon un premier aspect, l'invention concerne un procédé de multiplication et de culture de plantes de la famille des broméliacées, en particulier du genre ananas, pour obtenir des plantes ornementales en pot, caractérisé en ce qu'on fait croître la plante jusqu'à ce qu'il se forme un pédoncule floral, on coupe ce pédoncule floral et on fait enraciner le pédoncule floral portant le fruit, et cela dans un pot rempli de terre.

L'invention prévoit que la coupe du pédoncule floral a lieu à un stade de développement où celui-ci porte un fruit en formation.

On obtient ainsi une plante ornementale en pot différente des plantes en pot connues, puisqu'elle est constituée d'un pédoncule floral surmonté d'un fruit.

Contrairement aux plantes en pot connues, elle ne comporte ni tige, ni feuilles encombrantes et est plus facile à transporter, les quelques ébauches de feuilles engainantes n'étant pas gênantes.

Si l'aspect de cette plante en pot ressemble à celui de la fleur coupée traditionnelle, elle n'en constitue pas moins une plante pourvue de racines, dont la durée de conservation est beaucoup plus longue que celle de la fleur coupée.

Si les circonstances sont favorables, on peut même constater dans certains cas, et au bout de plusieurs mois, l'apparition de feuilles, par exemple à la place du fruit qui, entre-temps, se sera desséché.

Ce procédé de multiplication et de culture s'applique à toutes les plantes de la famille des broméliacées, en particulier à celles du genre ananas, mais aussi à celles de genres Vriesea, Guzmania, etc.

Conformément à une autre caractéristique de l'invention, on coupe le pédoncule floral à un stade de développement où la fleur, qui a donné naissance au fruit, est tombée et où il ne subsiste que les carpelles de la fleur qui constituent ensemble le fruit en formation. Le plus souvent, on coupe le pédoncule floral entre le 15ème et le 60ème jour après induction florale.

Ce sectionnement peut donc s'effectuer à un stade de maturation plus ou moins avancé du fruit, étant entendu qu'il est généralement préférable de l'effectuer à un stade précoce de la formation du fruit.

Selon une autre caractéristique de l'invention, le sectionnement du pédoncule floral s'effectue à un endroit quelconque en dessous du fruit et au-dessus de l'attache entre le pédoncule floral et la tige. On peut ainsi obtenir un pédondule floral de longueur désirée, en fonction des caractéristiques esthétiques et dimensionnelles que l'on désire donner à la plante ornementale.

Ce sectionnement s'effectue de préférence entre deux noeuds successifs du pédoncule floral ou, en variante, légèrement au-dessous du raccordement entre le pédoncule floral et la tige.

Pour faciliter l'enracinement du pédoncule floral dans son substrat nutritif, c'est-à-dire dans la terre du pot, on fait appel à des hormones végétales naturelles ou synthétiques.

On peut utiliser l'une quelconque des hormones connues à cet effet, en particulier celles du type des auxines ou des cytokines. Parmi celles-ci, on peut citer ı notamment : l'acide indolacétique, l'acide indolbutyrique, l'ace naphténacétique, la benzyladénine, la benzylaminopurine et leurs mélanges.

L'invention vise également les plantes ornementales en pot obtenues par la mise en oeuvre du procédé de multiplication et de culture défini précédemment.

Ces plantes ornementales ont des caractéristiques morphologiques bien précises qui les différencient des plantes ornementales de la famille des broméliacées connues jusqu'à présent.

Dans la description qui suit, donnée uniquement à titre d'exemple, on se réfère aux dessins annexés sur lesquels :

    - la figure 1 est une représentation simplifiée d'une plante du genre ananas portant un fruit en développement; et

    - la figure 2 est une représentation simplifiée, en partie en coupe, d'une plante ornementale en pot selon l'invention, obtenue après sectionnement du pédoncule floral de la plante représentée à la figure 1 et mise en pot.

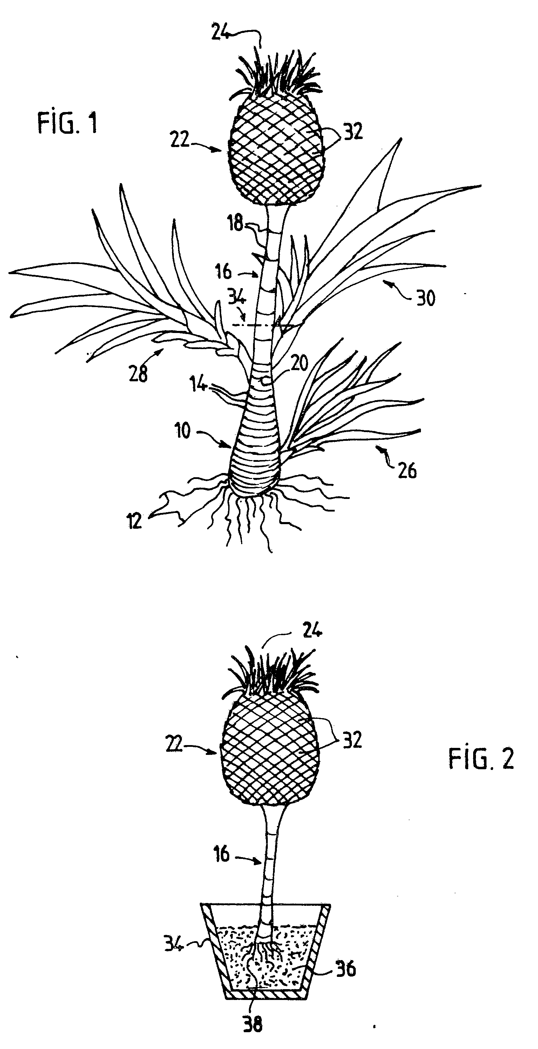

La plante représentée à la figure 1 possède une tige 10 qui est pourvue de racines 12 à sa base et qui présente des noeuds 14. De ces noeuds partent des feuilles, qui n'ont pas été représentées sur la figure 1 à des fins de simplification. Au-dessus de la tige 10, et prolongeant celle-ci, la plante possède un pédoncule floral 16 dont les noeuds 18 sont beaucoup plus espacés que les noeuds 14 de la tige 10. Le raccordement du pédoncule floral 16 avec la tige 10 se situe approximativement au niveau d'une attache 20. A son sommet, le pédoncule floral 16 porte un fruit 22, en l'espèce un ananas, qui est surmonté d'une couronne de feuilles 24 ou toupet.

La plante possède en outre un certain nombre de rejets tels que ceux montrés en 26, 28 et 30 qui se sont formés à partir de bourgeons ayant poussé sur la tige de la plante. Comme déjà indiqué, ces rejets sont après sélection séparés de la plantemère pour permettre ensuite la production d'autres plantes.

Pour la mise en oeuvre du procédé de l'invention, on fait croître la plante jusqu'à ce qu'il se forme le pédoncule floral 16 et on coupe ce pédoncule floral à un stade de développement où la fleur, qui a donné naissance à ce fruit, est tombée et où il ne subsiste que les carpelles 32 de la fleur qui constituent ensemble le fruit en formation et qui ont un aspect caractéristique d'écailles de forme hexagonale. Le plus souvent, on coupe le pédoncule floral entre le 15ème et le 60ème jour après induction florale.

La plante étant parvenue à ce stade de développement, on sectionne le pédoncule floral 16 à un endroit choisi pour obtenir un pédoncule floral de longueur désirée. Ce sectionnement s'effectue à un endroit quelconque en dessous du fruit 22 et audessus de l'attache 20 entre le pédoncule floral et la tige, de préférence entre deux noeuds 18 consécutifs. Dans le cas particulier de la figure 1, ce sectionnement s'effectue comme montré par la ligne interrompue 34.

Après sectionnement, et pour favoriser l'enracinement du pédoncule floral dans un pot, on utilise des hormones végétales naturelles ou synthétiques, de préférence du type des auxines ou des cytokinines. A titre d'exemples, on peut utiliser l'acide indolacétique, l'acide indolbutyrique, l'acide naphténacétique, la benzyladénine, la benzylaminopurine et leurs mélanges.

Le traitement s'effectue en trempant la base du pédoncule floral sectionné dans une solution de ces hormones végétales et/ ou en imprégnant le substrat, c'est-à-dire la terre du pot, avec cette même solution.

La figure 2 montre la plante ornementale obtenue après enracinement du pédoncule floral 16 dans un pot 34 rempli d'un substrat 36 approprié. Du fait de l'utilisation des hormones végétales précitées, des racines 38 se développent à la base du pédoncule floral préalablement sectionné et on obtient ainsi une plante ornementale en pot d'un type nouveau, qui est constituée essentiellement par le pédoncule floral 16 surmonté du fruit 22. Cette plante ornementale a une durée de conservation beaucoup plus longue que celle de la fleur coupée obtenue jusqu'à présent par sectionnement du pédoncule floral et conservation dans un vase rempli d'eau. Le fruit de la plante ornementale de l'invention peut ainsi se conserver plusieurs mois, surtout si le sectionnement du pédoncule floral a été effectué à un stade précoce de développement du fruit.

Si les circonstances sont favorables, la plante ornementale peut, après quelques semaines ou quelques mois, donner naissance à des feuilles qui viendront remplacer le fruit qui, entretemps, se sera desséché et aura été coupé.

## Revendications

1.- Procédé de multiplication de culture de plantes de la famille des broméliacées, en particulier du genre ananas, pour obtenir des plantes en pot, caractérisé en ce qu'on fait croitre la plante à la manière habituelle jusqu'à ce qu'il se forme un pédoncule floral, on coupe ce pédoncule floral et on le fait enraciner dans un pot empli de terre.

2. Procédé selon la revendication 1, caractérisé en ce qu'on coupe le pédoncule floral à un stade de développement où il porte un fruit en formation.

3.- Procédé selon la revendication 1 ou 2, caractérisé en ce que l'on coupe le pédoncule floral (16) à un stade de développement où la fleur, qui a donné naissance au fruit, est tombée et où il ne subsiste que les carpelles (32) de la fleur qui constituent ensemble le fruit (22) en formation.

4.- Procédé selon l'une des revendications 1 à 3, caractérisé en ce qu'on coupe le pédoncule floral (16) à un endroit situé en dessous du fruit (22) et au-dessus de l'attache (20) entre le pédoncule floral (16) et la tige (10), choisi pour obtenir un pédoncule floral de longueur désirée.

5.- Procédé selon l'une des revendications 1 à 4, caractérisé en ce que l'on coupe le pédoncule floral (16) entre deux noeuds (18) successifs.

6. Procédé selon l'une des revendications 1 à 3, caractérisé en ce que, en variante, le sectionnement a lieu légèrement au-dessous du raccordement entre le pédoncule floral et la tige.

7.- Procédé selon l'une des revendications 1 à 6, caractérisé en ce qu'on favorise l'enracinnement du pédoncule floral (16) en ajoutant à la terre des hormones végétales, naturelles ou synthétiques.

8.- Procédé selon la revendication 7, caractérisé en ce que ces hormones sont du type des auxines ou des cytokinines.

9.- Procédé selon la revendication 8, caractérisé en ce que ces hormones sont choisies parmi le groupe constitué par l'acide indolacétique, l'acide indolbutyrique, l'acide naphténacétique, la benzyladénine, la benzylaminopurine et leurs mélanges.

10.- Plante ornementale en pot obtenue par la mise en oeuvre du procédé selon l'une des revendications 1 à 5 et 7 à 9.

11. Plante ornementale en pot obtenue par la mise en oeuvre des revendications 6 à 9.

FIG. 1

FIG. 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-4 253 274  (GOLD)<br>* En entier *<br>--- | 1-5,7,8<br>,10,11 | A 01 H    5/02<br>A 01 G    7/00 |
| Y | WO-A-8 603 934  (CHAINTRON)<br>* Page 3, ligne 7 - page 5, ligne 13;<br>revendications 1-3,5,6; figures 2,4 *<br>----- | 1-5,7,8<br>,10,11 | |

**DOMAINES TECHNIQUES RECHERCHES (Int. Cl.4)**

A 01 H
A 01 G

Le présent rapport a été établi pour toutes les revendications

| Lieu de la recherche | Date d'achèvement de la recherche | Examinateur |
|---|---|---|
| LA HAYE | 06-12-1988 | DISSEN H.D. |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons
........................................................................................
& : membre de la même famille, document correspondant

EPO FORM 1503 03.82 (P0402)